# EUROPÄISCHE PATENTANMELDUNG

(11) **EP 3 875 979 A1**
(43) Veröffentlichungstag der Anmeldung: **08.09.2021**
(21) Anmeldenummer: 20161359.3
(22) Anmeldetag: 06.03.2020
(51) Int. Cl.: G01R 33/56, G01R 33/54, A61B 5/055, G01R 33/561, G06N 3/08

(54) **OPTIMIERTES VERFAHREN FÜR DYNAMISCHE KONTRASTVERSTÄRKENDE MAGNETRESONANZTOMOGRAPHIE**

(71) Anmelder: Bayer Aktiengesellschaft, 51373 Leverkusen (DE)
(72) Erfinder: Knobloch, Gesine, 10437 Berlin (DE); Ramos dos Santos, Thiago, 10717 Berlin (DE)
(74) Vertreter: BIP Patents

(57) **Zusammenfassung**

Die vorliegende Erfindung befasst sich mit der dynamischen kontrastverstärkenden Magnetresonanztomographie. Gegenstände der vorliegenden Erfindung sind ein Verfahren, eine Vorrichtung, ein System und ein Computerprogrammprodukt zur Ermittlung eines optimierten Zeitpunktes zum Starten der Aufnahme und Erfassung einer MRT-Aufnahme nach der Applikation eines Kontrastmittels. Ein weiterer Gegenstand der vorliegenden Erfindung betrifft ein Kontrastmittel zur Verwendung in einem MRT-Verfahren sowie ein Kit umfassend ein Kontrastmittel und ein Computerprogrammprodukt.

## Beschreibung

Die vorliegende Erfindung befasst sich mit der dynamischen kontrastverstärkenden Magnetresonanztomographie. Gegenstände der vorliegenden Erfindung sind ein Verfahren, eine Vorrichtung, ein System und ein Computerprogrammprodukt zur Ermittlung eines optimierten Zeitpunktes zum Starten der Aufnahme und Erfassung einer MRT-Aufnahme nach der Applikation eines Kontrastmittels. Ein weiterer Gegenstand der vorliegenden Erfindung betrifft ein Kontrastmittel zur Verwendung in einem MRT-Verfahren sowie ein Kit umfassend ein Kontrastmittel und ein Computerprogrammprodukt.

Die Magnetresonanztomographie, abgekürzt MRT oder MR (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz (HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer Tl-Verkürzung führen. Eine Verkürzung der T1-Zeit führt zu einer Zunahme der Signalintensität in T1-gewichteten MRT-Aufnahmen, eine Verkürzung der T2-Zeit führt zu einer Abnahme der Signalintensität in T2-gewichteten MRT-Aufnahmen.

Die Wirkung dieser Kontrastmittel ist indirekt, da das Kontrastmittel selbst kein Signal abgibt, sondern nur die Signalintensität der Wasserstoffprotonen in seiner Umgebung beeinflusst.

Ein Beispiel für ein superparamagnetisches Kontrastmittel sind Eisenoxidnanopartikel (SPIO, engl.: *superparamagnetic iron oxide*).

Beispiele für paramagnetische Kontrastmittel sind Gadolinium-Chelate wie Gadopentetat-Dimeglumin (Handelsname: Magnevist® u.a.), Gadobenat-Dimeglumin (Handelsname: Multihance®), Gadotersäure (Dotarem®, Dotagita®, Cyclolux®), Gadodiamid (Omniscan®), Gadoteridol (ProHance®) und Gadobutrol (Gadovist®).

Nach ihrem Verteilungsmuster im Gewebe können extrazelluläre, intrazelluläre und intravaskuläre Kontrastmittel unterschieden werden.

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Fokale Leberläsionen wie z.B. Zysten, Metastasen und der meisten Leberzellkarzinome enthalten keine/kaum Hepatozyten und nehmen das Kontrastmittel somit nicht oder nur kaum auf. Im Gegensatz zum umliegenden normalen Leberwegwebe werden dieses Läsionen nicht signalverstärkt dargestellt und werden damit besser erkennbar und lokalisierbar.

Beispiele für Kontrastmittel auf der Basis der Gadoxetsäure sind in US 6,039,931A beschrieben; sie sind kommerziell zum Beispiel unter den Markennamen Primovist® oder Eovist® erhältlich.

Ein weiteres Kontrastmittel mit einer geringeren Aufnahme in die Hepatozyten ist Gadobenate Dimeglumin (Multihance®).

Der kontrastverstärkende Effekt von Primovist®/Eovist® wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine hohe thermodynamische Stabilität aufweist. Der Ethoxybenzylrest (EOB) ist der Vermittler der hepatozytären Aufnahme des Kontrastmittels.

Primovist® kann zur Detektion und Differenzierung gutartiger und bösartiger Tumoren in der Leber eingesetzt werden. Die Blutversorgung des gesunden Lebergewebes erfolgt in erster Linie über die Pfortader (*Vena portae*), während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Bei der Differenzierung der Tumoren bzw. Läsionen nutzt man unter anderem die unterschiedliche Blutversorgung von Leber und Tumor und den zeitlichen Verlauf der Kontrastverstärkung. Im Fall von Primovist® nutzt man zusätzlich die unterschiedlichen Fähigkeiten der Zellen für die Aufnahme von Primovist®.

Die erzielte Kontrastverstärkung kann man in mindestens zwei Phasen unterteilen: in eine dynamische Phase (umfassend die so genannte arterielle Phase, portalvenöse Phase und Spätphase/Transitionalphase) und im Falle von Primovist® zusätzlich die hepatobiliäre Phase, in der bereits eine signifikante Aufnahme von Primovist® in die Hepatozyten stattgefunden hat.

Die zeitliche Verfolgung der Verteilung des Kontrastmittels über die dynamische Phase und die hepatobiliäre Phase bietet eine gute Möglichkeit der Detektion und Differentialdiagnose fokaler Leberläsionen. Die arterielle Phase ist von besonderer Bedeutung für die Differenzierung verschiedener fokaler Leberläsionen (z.B. Unterscheidung verschiedener Arten von Metastasen oder lebereigener Tumoren). So kann zum Beispiel die korrekte Diagnose des hepatozellulären Karzinoms (HCC) nur getroffen werden, wenn die Läsion ein eindeutiges arterielles Hyperenhancement (verstärkte arterielle Durchblutung mit Kontrastmittel) aufweist.

Die arterielle Phase ist jedoch vergleichsweise kurz; sie beträgt je nach Injektionsvolumen und Injektionsgeschwindigkeit nur etwa 10-20 Sekunden, wobei die durch das Kontrastmittel hervorgerufene Signalintensität in der arteriellen Phase einen klaren, nur wenige Sekunden dauernden Peak aufweist, ähnlich einer Pulswelle. Für einen optimalen arteriellen Kontrast in der Leber muss exakt jener Zeitpunkt mit dem MRT erfasst werden, in dem die arterielle Pulswelle mit dem Kontrastmittel durch das Lebergewebe fließt. In der Praxis weichen die Scan-Protokolle zum Erreichen dieses Ziels zum Teil stark voneinander ab. Üblich ist zum Beispiel die visuelle oder automatische (mittels *region of interest,* ROI) Detektion des Kontrastmittelbolus beim Erreichen der abdominellen Aorta auf Höhe des Zwerchfells. Die Ankunft des Bolus löst dann (manuell oder automatisch) ein zeitverzögertes Starten des MRT-Scans der Leber aus. Diese zeitliche Verzögerung wird jedoch fix festgelegt, sie ist nicht patientenindividuell und sie unterscheidet sich oft stark von Klinik zu Klinik. Noch einfachere Verfahren verwenden eine gänzlich fixierte Start-Verzögerung (z.B. Start des Scans immer starr 25 s nach Injektionsbeginn), was je nach Kreislaufzeit des Patienten zu suboptimalen Bildergebnissen der arteriellen Phase führt.

Die diagnostische Genauigkeit des Leber MRTs, die maßgeblich von der Qualität der arteriellen Phase abhängt, unterscheidet sich deswegen teils deutlich je nach verwendetem Scan-Protokoll. Es mangelt somit an standardisierten Verfahren, die unter Berücksichtigung der patientenindividuellen Kreislaufzeit ein Starten der MRT-Aufnahme während des optimalen arteriellen Zeitfensters im Leberparenchym induzieren.

Aufgrund der Formulierung von Primovist® (75% weniger Gadolinium pro applizierter Standarddosis, im Vergleich zu den meisten GBCAs (*Gadolinium-based contrast agents*) und 50% geringeres Flüssigkeitsvolumen) und sich der daraus ergebenden geringen Signalstärke und kürzeren Boluslänge, ist es für dieses Kontrastmittel besonders kritisch und wichtig, den optimalen Enhancement Peak innerhalb des Leberparenchyms abzupassen. Aufgrund der geringen Boluslänge besteht außerdem die Gefahr, dass die Bildakquisition der arteriellen Phase entweder zu früh oder zu spät gestartet und somit die Leberläsion nicht im optimalen Zeitfenster erfasst wird. Diese Fehlerquelle in der Bildgebung kann zu Fehldiagnosen führen.

Um die arterielle Phase z.B. in der Leber optimal zu erfassen, sollte eine erste MRT-Aufnahme nicht etwa beim maximalen Enhancement der Leberarterie oder abdominellen Aorta, sondern erst (Sekunden) später - während des maximalen Enhancments des Leberparenchyms aufgenommen werden.

Die gleichen Überlegungen gelten auch für andere Untersuchungsbereiche.

Ziel der Erfindung ist daher ein patienten-individualisiertes, standardisiertes und automatisiertes Starten des MRT-Scans während eines optimalen Zeitfensters.

Ausgehend vom beschriebenen Stand der Technik stellt sich einem Fachmann daher die technische Aufgabe, den optimalen Zeitpunkt zum Starten der Erzeugung einer MRT-Aufnahme nach einer Applikation eines Kontrastmittels zu identifizieren.

Diese Aufgabe wird durch die Gegenstände der unabhängigen Patentansprüche gelöst. Bevorzugte Ausführungsformen der vorliegenden Erfindung finden sich in den abhängigen Patentansprüchen, in dieser Beschreibung und in den Zeichnungen.

Ein erster Gegenstand der vorliegenden Erfindung ist ein Verfahren umfassend die Schritte:
- Empfangen eines ersten Signals, wobei das erste Signal einen ersten Zeitpunkt kennzeichnet, wobei zum ersten Zeitpunkt einem Untersuchungsobjekt ein Kontrastmittel in Form eines Bolus verabreicht wird,
- Empfangen eines zweiten Signals, wobei das zweite Signal einen zweiten Zeitpunkt kennzeichnet, wobei zum zweiten Zeitpunkt der Bolus einen Kontrollbereich erreicht,
- Berechnen eines dritten Zeitpunkts auf Basis des ersten Zeitpunkts und des zweiten Zeitpunkts, wobei der Bolus zum dritten Zeitpunkt einen Untersuchungsbereich erreicht,
- Ausgeben eines Startsignals zum dritten Zeitpunkt und/oder zu einem vierten Zeitpunkt, wobei der vierte Zeitpunkt in einem definierten zeitlichen Abstand zum dritten Zeitpunkt liegt,
- Starten der Erfassung einer MRT-Aufnahme von dem Untersuchungsbereich zum dritten oder vierten Zeitpunkt.

Ein weiterer Gegenstand der vorliegenden Erfindung ist eine Vorrichtung umfassend:
- eine Empfangseinheit,
- eine Steuer- und Recheneinheit, und
- eine Ausgabeeinheit
   - wobei die Steuer- und Recheneinheit konfiguriert ist, die Empfangseinheit zu veranlassen, ein erstes Signal zu empfangen, wobei das erste Signal einen ersten Zeitpunkt kennzeichnet, wobei zum ersten Zeitpunkt einem Untersuchungsobjekt ein Kontrastmittel in Form eines Bolus verabreicht wird,
   - wobei die Steuer- und Recheneinheit konfiguriert ist, die Empfangseinheit zu veranlassen, ein zweites Signal zu empfangen, wobei das zweite Signal einen zweiten Zeitpunkt kennzeichnet, wobei zum zweiten Zeitpunkt der Bolus einen Kontrollbereich erreicht,
   - wobei die Steuer- und Recheneinheit konfiguriert ist, auf Basis des ersten Zeitpunkts und des zweiten Zeitpunkts einen dritten Zeitpunkt zu berechnen, wobei der Bolus zum dritten Zeitpunkt einen Untersuchungsbereich erreicht,
   - wobei die Steuer- und Recheneinheit konfiguriert ist, die Ausgabeeinheit zu veranlassen, ein Startsignal zum dritten Zeitpunkt und/oder zu einem vierten Zeitpunkt auszugeben, wobei der vierte Zeitpunkt in einem definierten zeitlichen Abstand zum dritten Zeitpunkt liegt.

Ein weiterer Gegenstand ist ein System umfassend:
- einen Injektor, wobei der Injektor konfiguriert ist, einem Untersuchungsobjekt Kontrastmittel in Form eines Bolus zu einem ersten Zeitpunkt zu verabreichen,
- eine Sensoreinheit, wobei die Sensoreinheit konfiguriert ist, das Eintreffen des Bolus in einem Kontrollbereich zu einem zweiten Zeitpunkt zu erfassen,
- eine Steuer- und Recheneinheit, wobei die Steuer- und Recheneinheit konfiguriert ist, auf Basis des ersten Zeitpunkts und des zweiten Zeitpunkts einen dritten Zeitpunkt zu berechnen, wobei der Bolus zum dritten Zeitpunkt einen Untersuchungsbereich erreicht,
- eine Aufnahmeeinheit für MRT-Aufnahmen, wobei die Aufnahmeeinheit konfiguriert ist, die Erzeugung einer MRT-Aufnahme von dem Untersuchungsbereich zum dritten Zeitpunkt oder zu einem vierten Zeitpunkt zu starten, wobei der vierte Zeitpunkt in einem definierten zeitlichen Abstand zum dritten Zeitpunkt liegt.

Ein weiterer Gegenstand der vorliegenden Erfindung ist ein Computerprogrammprodukt umfassend einen Datenträger, auf dem ein Computerprogramm gespeichert ist, wobei das Computerprogramm in einen Arbeitsspeicher eines Computers geladen werden kann und dort den Computer dazu veranlasst, folgende Schritte ausführen:
- Empfangen eines ersten Signals, wobei das erste Signal einen ersten Zeitpunkt kennzeichnet, wobei zum ersten Zeitpunkt einem Untersuchungsobjekt ein Kontrastmittel in Form eines Bolus verabreicht wird,
- Empfangen eines zweiten Signals, wobei das zweite Signal einen zweiten Zeitpunkt kennzeichnet, wobei zum zweiten Zeitpunkt der Bolus einen Kontrollbereich erreicht,
- Berechnen eines dritten Zeitpunkts auf Basis des ersten Zeitpunkts und des zweiten Zeitpunkts, wobei der Bolus zum dritten Zeitpunkt einen Untersuchungsbereich erreicht,
- Ausgeben eines Startsignals zum dritten Zeitpunkt und/oder zu einem vierten Zeitpunkt, wobei der vierte Zeitpunkt in einem definierten zeitlichen Abstand zum dritten Zeitpunkt liegt,
- Starten der Erfassung einer MRT-Aufnahme von dem Untersuchungsbereich zum dritten oder vierten Zeitpunkt.

Ein weiterer Gegenstand der vorliegenden Erfindung ist die Verwendung eines Kontrastmittels in einem MRT-Verfahren, wobei das MRT-Verfahren die folgenden Schritte umfasst:
- Applizieren des Kontrastmittels in Form eines Bolus zu einem ersten Zeitpunkt,
- Erfassen des Eintreffens des Bolus in einem Kontrollbereich,
- Berechnen eines Startzeitpunkts anhand der Zeitspanne zwischen dem Applizieren des Kontrastmittels und dem Eintreffen des Bolus in dem Kontrollbereich,
- Starten der Erzeugung einer MRT-Aufnahme von einem Untersuchungsbereich zum Startzeitpunkt.

Ein weiterer Gegenstand ist ein Kontrastmittel zur Verwendung in einem MRT-Verfahren, wobei das MRT-Verfahren die folgenden Schritte umfasst:
- Applizieren des Kontrastmittels in Form eines Bolus zu einem ersten Zeitpunkt,
- Erfassen des Eintreffens des Bolus in einem Kontrollbereich,
- Berechnen eines Startzeitpunkts anhand der Zeitspanne zwischen dem Applizieren des Kontrastmittels und dem Eintreffen des Bolus in dem Kontrollbereich,
- Starten der Erzeugung einer MRT-Aufnahme von einem Untersuchungsbereich zum Startzeitpunkt.

Ein weiterer Gegenstand ist ein Kit umfassend ein Kontrastmittel und ein erfindungsgemäßes Computerprogrammprodukt.

Die Erfindung wird nachstehend näher erläutert, ohne zwischen den Erfindungsgegenständen (Verfahren, Vorrichtung, System, Computerprogrammprodukt, Verwendung, Kontrastmittel zur Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, Vorrichtung, System, Computerprogrammprodukt, Verwendung, Kontrastmittel zur Verwendung, Kit) sie erfolgen.

Ausgangspunkt der vorliegenden Erfindung ist ein Untersuchungsobjekt. Das "Untersuchungsobjekt" ist üblicherweise ein Lebewesen, vorzugsweise ein Säugetier, ganz besonders bevorzugt ein Mensch.

Ein Teil des Untersuchungsobjekts, der Untersuchungsbereich, soll einer kontrastverstärkten Magnetresonanztomographie-Untersuchung unterzogen werden.

Der "Untersuchungsbereich", auch Aufnahmevolumen (engl.: *field of view,* FOV) genannt, stellt insbesondere ein Volumen dar, welches in den Magnetresonanzaufnahmen abgebildet wird. Der Untersuchungsbereich wird typischerweise durch einen Radiologen, beispielsweise auf einer Übersichtsaufnahme (engl.: *localizer*/*scout*) festgelegt. Selbstverständlich kann der Untersuchungsbereich alternativ oder zusätzlich auch automatisch, beispielsweise auf Grundlage eines ausgewählten Protokolls, festgelegt werden. In einer bevorzugten Ausführungsform der vorliegenden Erfindung umfasst der Untersuchungsbereich zumindest einen Teil der Leber des Untersuchungsobjekts.

Dem Untersuchungsobjekt wird ein Kontrastmittel verabreicht. Das Kontrastmittel wird dem Untersuchungsobjekt an einer Applikationsstelle zu einem Applikationszeitpunkt verabreicht. Das Kontrastmittel erreicht nach einer Zeitspanne den Untersuchungsbereich und führt hier zu einer Kontrastverstärkung. Das Kontrastmittel wird vorzugsweise intravenös als Bolus, gewichtsadaptiert verabreicht. In einer bevorzugten Ausführungsform wird das Kontrastmittel in eine Armvene des Untersuchungsobjekts injiziert.

Unter einem "Kontrastmittel" wird ein Stoff oder Stoffgemisch verstanden, dessen Anwesenheit in einer Magnetresonanzmessung zu einem veränderten Signal führt. Vorzugsweise führt das Kontrastmittel zu einer Verkürzung der T1-Relaxationszeit und/oder der T2-Relaxationszeit.

Vorzugsweise ist das Kontrastmittel ein hepatobiliäres Kontrastmittel wie beispielsweise Gd-EOB-DTPA oder Gd-BOPTA.

In einer besonders bevorzugten Ausführungsform handelt es sich bei dem Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Der Zeitpunkt, zu dem das Kontrastmittel verabreicht wird, wird erfasst. Dieser Zeitpunkt wird in dieser Beschreibung auch als "der erste Zeitpunkt" oder "Applikationszeitpunkt" bezeichnet. Dabei kann der erste Zeitpunkt (Applikationszeitpunkt) derjenige Zeitpunkt sein, zu dem die Applikation des Kontrastmittels beginnt, es kann derjenige Zeitpunkt sein, zu dem die Applikation des Kontrastmittels beendet ist, es kann derjenige Zeitpunkt sein, zu dem ein definierter Bruchteil (z.B. ein Viertel oder ein Drittel oder die Hälfte oder zwei Drittel oder drei Viertel oder ein anderer Bruchteil) der Menge des zu verabreichenden Kontrastmittels verabreicht ist, oder es kann sich um einen anderen Zeitpunkt handelt, der mit der Applikation des Kontrastmittels in einem definierten Zusammenhang steht. Wichtig ist lediglich, dass zur Ausführung der Erfindung der Applikationszeitpunkt stets gleich definiert und eindeutig bestimmbar. Insbesondere dann, wenn zur Berechnung des optimierten Zeitpunkts zum Starten der Erzeugung einer MRT-Aufnahme ein mittels maschinellen Lernens erzeugtes Vorhersagemodell verwendet wird, muss dem maschinellen Lernen und der Berechnung des Startzeitpunktes derselbe Applikationszeitpunkt zugrunde gelegt werden.

Es ist denkbar, dass der erste Zeitpunkt willkürlich auf den Zeitpunkt t=0 festgesetzt wird. Alle übrigen Zeitpunkte und Zeitspannen, die im Rahmen der Ausführung der vorliegenden Erfindung erfasst und/oder ermittelt werden, orientieren sich dann an diesem Nullpunkt (Ursprung). Anders gesagt definiert der Applikationszeitpunkt den Start- oder Bezugspunkt für die Erfassung und/oder Ermittlung weiterer Zeitpunkte und/oder Zeitspannen.

Das als Bolus vorzugsweise intravenös verabreichte Kontrastmittel wird durch das Herz des Untersuchungsobjekts in dem Untersuchungsobjekt in Richtung des Untersuchungsbereichs transportiert. Auf diesem Weg erreicht es zu einem zweiten Zeitpunkt, der zeitlich auf den Applikationszeitpunkt folgt, einen Kontrollbereich. Der Kontrollbereich ist, wie auch der Untersuchungsbereich, ein Volumen innerhalb des Untersuchungsobjekts, durch das der Bolus hindurchtritt und/oder in dem sich der Bolus verteilt. Der zweite Zeitpunkt, in dieser Beschreibung auch als Kontrollzeitpunkt bezeichnet, kann derjenige Zeitpunkt sein, zu dem der Bolus in den Kontrollbereich eintritt, zu dem der Bolus aus dem Kontrollbereich austritt oder zu dem ein definierter Bruchteil des Kontrastmittels in dem Kontrollbereich vorhanden ist. Zur Detektion von Kontrastmittel im Kontrollbereich dient eine Sensoreinheit, welche konfiguriert ist, die Anwesenheit von Kontrastmittel im Kontrollbereich zu erkennen. Eine solche Sensoreinheit weist üblicherweise eine gewisse Messschwelle auf: es ist eine Mindestmenge an Kontrastmittel in dem Kontrollbereich nötig, um mit der Sensoreinheit die Anwesenheit von Kontrastmittel erkennen zu können. Der Kontrollzeitpunkt kann derjenige Zeitpunkt sein, bei dem die Messschwelle gerade überschritten ist. Zu diesem Zeitpunkt ist also gerade so viel Kontrastmittel in dem Kontrollbereich vorhanden, dass der Sensor die Anwesenheit von Kontrastmittel erfassen kann. Der Kontrollzeitpunkt kann aber auch derjenige Zeitpunkt sein, zu dem die Messchwelle gerade wieder unterschritten wird, wenn das Kontrastmittel den Kontrollbereich wieder verlässt. In einer bevorzugten Ausführungsform ist der Kontrollzeitpunkt derjenige Zeitpunkt, bei dem das durch Kontrastmittel in dem Kontrollvolumen erzeugte Signal ein Maximum oder Minimum erreicht (je nachdem, ob das jeweils verwendete Kontrastmittel zu einer Verstärkung der Abschwächung der Signalintensität führt).

Bei dem Kontrollbereich kann es sich um einen definierten Abschnitt (Volumen) in einer Vene oder einer Arterie des Untersuchungsobjekts handeln, durch das Kontrastmittel auf dem Weg von der Applikationsstelle zum Untersuchungsbereich hindurchtritt. Denkbar ist auch, dass es sich bei dem Kontrollbereich um das Herz oder eine Herzkammer handelt.

Der Kontrollbereich wird vorzugsweise so gewählt, dass er einer messtechnischen Ermittlung von Kontrastmittel in dem Kontrollbereich zugänglich ist.

Der Kontrollbereich wird ferner vorzugsweise so gewählt, dass auf der einen Seite die Zeitspanne zwischen dem Applikationszeitpunkt und dem Kontrollzeitpunkt möglichst groß ist (um Fehler infolge von Messungenauigkeiten zu minimieren), auf der anderen Seite die Zeitspanne zwischen dem Kontrollzeitpunkt und dem Zeitpunkt, zu dem das Kontrastmittel den Untersuchungsbereich erreicht (in dieser Beschreibung als dritter Zeitpunkt), jedoch groß genug ist, um den dritten Zeitpunkt (und ggf. einen vierten Zeitpunkt) berechnen und alle notwendigen Vorbereitungen zum Start der Erzeugung einer MRT-Aufnahme des Untersuchungsbereichs treffen zu können. Vorzugsweise ist die Zeitspanne zwischen dem Applikationszeitpunkt und dem Kontrollzeitpunkt größer als die Zeitspanne zwischen dem Kontrollzeitpunkt und dem dritten Zeitpunkt. Vorzugsweise beträgt die Zeitspanne zwischen dem Applikationszeitpunkt und dem Kontrollzeitpunkt mindestens das Anderthalbfache bis Zweifache der Zeitspanne zwischen dem Kontrollzeitpunkt und dem dritten Zeitpunkt.

Die Sensoreinheit umfasst mindestens einen Sensor, der die Anwesenheit von Kontrastmittel in dem Kontrollbereich detektieren kann und vorzugsweise auch die Menge an Kontrastmittel in dem Kontrollbereich zumindest semiquantitativ bestimmen kann (um zumindest ein Maximum von Kontrastmittel in dem Kontrollbereich erkennen zu können).

Zur Detektion von Kontrastmittel im Kontrollbereich kann man sich die magnetischen Eigenschaften des Kontrastmittels zu Nutze machen.

In einer bevorzugten Ausführungsform handelt es sich bei der Sensoreinheit um diejenige Einheit, die auch zur Erzeugung von MRT-Aufnahmen des Untersuchungsbereichs eingesetzt wird. Dabei kommt es bei der Detektion von Kontrastmittel in dem Kontrollbereich nicht darauf an, eine hohe räumliche Auflösung zu erzielen; viel wichtiger ist es, Änderungen der Menge an Kontrastmittel in dem Kontrollbereich möglichst schnell (mit einer zeitlichen Auflösung von beispielsweise mindestens einer Sekunde) zu erreichen. Hierzu können beispielsweise Verfahren der Echtzeit-Magnetresonanztomographie (Echtzeit-MRT oder auch MR-Fluoroskopie) verwendet werden, die eine hohe zeitliche Auflösung auf Kosten einer geringeren räumlichen Auflösung bieten (siehe z.B. US-Patent Nr. 4.830.012). Grundlage der Echtzeit-MRT sind sehr schnelle Messsequenzen, die eine Bildaufnahme mit einer hohen zeitlichen Auflösung zulassen. Typische Beispiele hierfür sind schnelle Gradientenecho-Sequenzen (z.B. die FLASH-Sequenz), die TrueFISP-Technik oder schnelle nichtsegmentierte Fast-/Turbo-Spin-Echo-Verfahren. Eine besonders schnelle MRT-Technik kombiniert eine FLASH-Sequenz mit erheblich unterabgetasteten radialen Trajektorien und einem iterativen Rekonstruktionsverfahren, das die Bildberechnung als Lösung eines nichtlinearen inversen Problems mit zeitlicher Regularisierung definiert (M. Uecker et al.: Real-time magnetic resonance imaging at a resolution of 20 ms, NMR in Biomedicine 23: 986-994 (2010), doi:10.1002/nbm.1585). Auf diese Weise wird eine zeitliche Auflösung 10 bis 40 Millisekunden pro Bild erreicht.

Ein weiteres Verfahren zur Erhöhung der zeitlichen Auflösung von MRT-Aufnahmen wird als "Schlüsselloch"-Bildgebung bezeichnet. Wie z.B. von R. A. Jones et al. in "Dynamic, Contrast Enhanced, NMR Perfusion Imaging of Regional Cerebral Ischaemia In Rats Using K-Space Substitution", SMR Eleventh Annual Meeting 1992, Abstr. 1138, beschrieben ist, wird eine Folge von Bildern während einer dynamischen Studie, bei der ein Kontrastmittel in das Untersuchungsobjekt injiziert wird, gewonnen. Das erste Bild in der Folge ist ein Bezugsbild, in dem alle Phasencodierungsansichten (d.h. 128 Ansichten) erfasst werden. Nachfolgende Bilder werden allerdings nur durch Gewinnung der zentralen Ansichten (d. h. der 32 zentralen Ansichten) erzeugt. Diese Schlüssellochabtastungen können viel schneller als vollständige Abtastungen gewonnen werden, wobei die Geschwindigkeitsrate proportional erhöht ist. Die Schlüssellochbilder werden unter Verwendung der neuesten k-Raum-Ansichten in Kombination mit den äußeren, peripheren k-Raum-Ansichten aus der Bezugsabtastung rekonstruiert.

Aus dem ersten Zeitpunkt (Applikationszeitpunkt) und dem zweiten Zeitpunkt (Kontrollzeitpunkt) wird der Zeitpunkt berechnet (vorhergesagt) zu dem das Kontrastmittel den Untersuchungsbereich erreicht (dritter Zeitpunkt). Dabei kann dieser dritte Zeitpunkt (analog zu dem ersten und dem zweiten Zeitpunkt) derjenige Zeitpunkt sein, zu dem die Messchwelle überschritten wird, unterschritten wird oder das vom Kontrastmittel hervorgerufene Signal ein Maximum oder ein Minimum durchläuft.

Aus dem ersten Zeitpunkt und dem zweiten Zeitpunkt lässt sich die Zeitspanne berechnen, die das Kontrastmittel benötigt, um von der Applikationsstelle zum Kontrollbereich zu gelangen. Aus dieser Zeitspanne lässt sich eine Kreislaufzeit für das Untersuchungsobjekt berechnen. Die Kreislaufzeit ist die Zeit, die eine Testsubstanz (im vorliegenden Fall das Kontrastmittel) für das Passieren einer Teststrecke des Kreislaufsystems benötigt. Aus der Kreislaufzeit wiederum lässt sich die Zeitspanne berechnen, die das Kontrastmittel benötigt, um den Untersuchungsbereich zu erreichen. Damit ergibt sich der dritte Zeitpunkt, zu dem das Kontrastmittel den Untersuchungsbereich erreichen wird.

Die Berechnung des dritten Zeitpunkts kann beispielsweise mit einem Modell des Kreislaufsystems erfolgen. Solche Modelle sind im Stand der Technik ausgiebig beschrieben (siehe z.B. WO2006/102597A2, EP2042100A2, Math Ind Case Stud. 2017; 8(1): 2 (doi: 10.1186/s40929-017-0011-1), Biomech Model Mechanobiol 2018 (17) 1217-1242 (doi: 10.1007/s10237-018-1024-9).

In einer bevorzugten Ausführungsform erfolgt die Berechnung des dritten Zeitpunkts mittels eines Regressionsmodells, das mittels maschinellem Lernen vorzugsweise in einem überwachten Lernverfahren anhand von Referenzdaten trainiert worden ist, anhand eines Applikationszeitpunkts und eines Kontrollzeitpunkts oder der Zeitspanne zwischen dem Kontrollzeitpunkt und dem Applikationszeitpunkt und ggf. anhand weiterer Inputparameter den dritten Zeitpunkt zu berechnen.

In einer bevorzugten Ausführungsform wird das Regressionsmodell mit einem Random-Forest-Verfahren trainiert. Ein Random-Forest-Verfahren ist ein Klassifikations- oder Regressionsverfahren, das aus mehreren unkorrelierten Entscheidungsbäumen besteht. Alle Entscheidungsbäume sind unter einer bestimmten Art von Randomisierung während des Lernprozesses gewachsen. Details zur Erzeugung eines Regressionsmodells mittels eines Random-Forrest-Verfahrens sind ausgiebig im Stand der Technik beschrieben (siehe z.B.: C. Sheppard: Tree-basedMachine Learning Algorithms: Decision Trees, Random Forests, and Boosting, CreateSpace Independent Publishing Platform 2017, ISBN: 978-1-9758-6097-4; T. W. Miller: Modeling Techniques in Predictive Analytics, Pearson Education, Inc., 2015, ISBN: 978-0-13-389206-2; P. Cichosz et al: Data Mining Algorithms, Wiley 2015, ISBN: 978-1-1183-3258-0).

In einer weiteren bevorzugten Ausführungsform ist oder umfasst das Regressionsmodell ein künstliches neuronales Netz. Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von Werten von Inputparametern wie beispielsweise dem ersten Zeitpunkt und dem zweiten Zeitpunkt und/oder der Zeitspanne zwischen dem ersten Zeitpunkt und dem zweiten Zeitpunkt. In einem solchen Netzwerk dient das mindestens eine Ausgangsneuron dazu, den dritten Zeitpunkt und/oder den vierten Zeitpunkt auszugeben. Die Verarbeitungselemente der Schichten zwischen den Eingangsneuronen und dem mindestens einen Ausgangsneuron sind in einem vorbestimmten Muster mit vorbestimmten Verbindungsgewichten miteinander verbunden.

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabevektoren auf gegebene Ausgabevektoren angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte .

Im trainierten Zustand enthalten die Verbindungsgewichte zwischen den Verarbeitungselementen Informationen bezüglich der Beziehung zwischen den Inputparametern und der Ausgabe (dem dritten und/oder vierten Zeitpunkt), die beispielsweise verwendet werden können, um den dritten und/oder vierten Zeitpunkt für ein neues Untersuchungsobjekt, dem ein Kontrastmittel verabreicht worden ist, vorherzusagen.

Eine Kreuzvalidierungsmethode kann verwendet werden, um die Daten in Trainings- und Validierungsdatensätze aufzuteilen. Der Trainingsdatensatz wird beim Backpropagations-Training der Netzwerkgewichte verwendet. Der Validierungsdatensatz wird verwendet, um die Vorhersagegenauigkeit des trainierten Netzwerks zu ermitteln.

Details zur Erzeugung und zum Trainieren künstlicher neuronaler Netze sind beispielsweise beschrieben in: G. Ciaburro et al.: Neural Networks with R, Packt Publishing 2017, ISBN: 978-1-78839-787-2; T. Rashid: Make Your Own Neural Network, O'Reilly 2016, ISBN: 978-1530826605.

Der Berechnung der dritten Zeitspanne (und/oder der vierten Zeitspanne) können die folgenden Inputparameter zugrunde gelegt werden:
- Applikationszeitpunkt und Kontrollzeitpunkt bzw. die Zeitspanne zwischen dem Applikationszeitpunkt und dem Kontrollzeitpunkt,
- Größe und Gewicht des Untersuchungsobjekts
- Alter des Untersuchungsobjekts
- Körperzusammensetzung des Untersuchungsobjekts (z.B. gemessen mittels bioelektrischer Impedanzanalyse, beispielsweise in Form der Fettmasse (*fat mass),* des Körperwassers *(total body water*), der Muskelmasse, der fettfreien Masse (*fat free mass*), der Körperzellmasse (body cell mass), der extrazellulären Masse (extracellular mass) und/oder der Magermasse (lean body mass)
- Menge des applizierten Kontrastmittels
- Art des applizierten Kontrastmittels (z.B. in Form eines Produktnamens, einer chemischen Formel und/oder dergleichen)
- Herzeigenschaften: z.B. Größe, Durchmesser, Schlagfrequenz, Schlagvolumen, Signalgebung des Herzmuskels
- Voraufnahmen aus anderen bildgebenden Verfahren (CT, MRT, kontrastverstärkter Ultraschall), die Aufschluss über die Zirkulationszeit des Untersuchungsobjektes zulassen oder Veränderungen im Ziel-Untersuchungsgebiet abbilden, die zu Verzögerungen in der Organperfusion führen könnten
- Hinweise auf makro- und mikroangiopatische Veränderungen aus bildgebenden Verfahren oder sonstigen Vorberichten aus der Krankengeschichte, die zu einer Verzögerung der Organperfusion führen könnten

Der berechnete dritte Zeitpunkt ist nicht notwendigerweise derjenige Zeitpunkt, zu dem die Erzeugung einer MRT-Aufnahme (mit vorzugsweise einer für diagnostische Zwecke ausreichend hohen räumlichen Auflösung des Untersuchungsbereichs) starten sollte. Es kann beispielsweise sein, dass der dritte Zeitpunkt als derjenige Zeitpunkt definiert ist, bei dem das Signal, das von dem Kontrastmittel im Kontrollbereich verursacht oder beeinflusst wird, ein Maximum oder Minimum durchläuft. Es kann sein, dass dieser Zeitpunkt zu spät für den Start der Erzeugung einer MRT-Aufnahme ist. Daher wird ein vierter Zeitpunkt definiert, der in dieser Beschreibung auch als Startzeitpunkt bezeichnet wird. Der vierte Zeitpunkt (Startzeitpunkt) ist derjenige Zeitpunkt, zu dem die Erzeugung einer MRT-Aufnahme gestartet werden soll (um eine für diagnostische Zwecke optimale MRT-Aufnahme zu erhalten). Dieser vierte Zeitpunkt liegt in einem definierten zeitlichen Abstand zum dritten Zeitpunkt. Der vierte Zeitpunkt kann zeitlich vor dem dritten Zeitpunkt (negative Werte für den zeitlichen Abstand) oder zeitlich nach dem dritten Zeitpunkt (positive Werte für den zeitlichen Abstand) liegen. Ferner kann der zeitliche Abstand zwischen dem dritten Zeitpunkt und dem vierten Zeitpunkt auch Null betragen.

Zur Festlegung des zeitlichen Abstands zwischen dem dritten Zeitpunkt und dem vierten Zeitpunkt können Parameter wie beispielsweise die geplante Aufnahmetechnik, die benötigte Zeit für Atemkommandos und/oder dergleichen berücksichtigt werden. Vorzugsweise werden zur Berechnung des vierten Zeitpunkts (auch) spezifische MRT-Sequenzparameter berücksichtigt. So kann sich -je nach Auslese des K-Raumes - in der einen oder der anderen Sequenz der Beginn des optimalen Akquisitionsfensters für die arterielle Phase verschieben (z.B. kann bei einer zentrischen Auslese des K-Raumes früher gestartet werden, bei einer kartesischen (Zeile für Zeile, linearen) Auslese muss früher gestartet werden, damit die Mitte der K-Raum Auslese mit dem Maximum Kontrast in der Leber zusammentrifft). Dem Fachmann (Radiologen) ist bekannt, wie er einen optimalen vierten Zeitpunkt für das jeweils verwendete MRT-Aufnahmegerät und die verwendete Messsequenz aus dem dritten Zeitpunkt errechnen kann.

Es ist denkbar, dass das Modell zur Berechnung des dritten Zeitpunkts konfiguriert ist, den vierten Zeitpunkt direkt (ohne "Umweg" über den dritten Zeitpunkt) zu berechnen. Es ist beispielsweise denkbar, dass ein Modell anhand von Referenzdaten trainiert worden ist, auf Basis des Applikationszeitpunkts und des Kontrollzeitpunkts bzw. der Zeitspanne dazwischen und ggf. weiterer Inputgrößen einen optimalen Zeitpunkt zum Starten der Erzeugung einer MRT-Aufnahme zu berechnen. In ein solches Training können beispielsweise MRT-Aufnahmen als Referenzdaten einbezogen worden sein, deren Erzeugung in unterschiedlichen Abständen zum dritten Zeitpunkt gestartet worden ist, und deren Eignung für diagnostische Zwecke beispielsweise durch einen Radiologen bewertet worden ist.

Es ist denkbar, dass mehrere Modelle vorhanden sind, wobei ein oder mehrere Inputparameter bestimmen, welches Modell zur Berechnung des dritten und/oder vierten Zeitpunkts verwendet wird. Es ist beispielsweise denkbar, dass es für verschiedene Arten von Kontrastmitteln verschiedene Modelle gibt. Zu Beginn einer MRT-Untersuchung wird das jeweils verwendete Kontrastmittel angegeben (spezifiziert). Anhand des angegebenen Kontrastmittels wird automatisch das jeweilige Modell ausgewählt, das konfiguriert (z.B. trainiert) ist, für das jeweilige Kontrastmittel den dritten und/oder vierten Zeitpunkt zu berechnen. In analoger Weise ist es denkbar, dass es verschiedene Modelle für verschiedene Kontrollbereiche, Untersuchungsbereiche, Applikationsstellen, Typen von Untersuchungsobjekten und/oder dergleichen gibt.

Es ist denkbar, dass die erfindungsgemäße Vorrichtung, das erfindungsgemäße System und/oder das erfindungsgemäße Computerprogrammprodukt konfiguriert sind, bei Erreichen des vierten Zeitpunkts ein Signal (Startsignal) zum Starten der Erzeugung einer MRT-Aufnahme zu erzeugen. Das Signal initiiert (triggert) in einem solchen Fall den Start der Erzeugung einer MRT-Aufnahme durch ein MRT-Aufnahmegerät (das ein Bestandteil des erfindungsgemäßen Systems sein kann).

In einer bevorzugten Ausführungsform sind die erfindungsgemäße Vorrichtung, das erfindungsgemäße System und das erfindungsgemäße Computerprogrammprodukt so konfiguriert, dass sie einen dritten und/oder einen vierten Zeitpunkt für verschiedene Applikationsstellen, Applikationsarten, Mengen an applizierten Kontrastmittel, Kontrollbereiche, Untersuchungsbereiche und/oder Arten und/oder Mengen von Kontrastmittel berechnen können. In einem ersten Schritt werden demnach die variablen Größen durch einen Nutzer (z.B. einen Radiologen) oder automatisiert spezifiziert. Spezifizieren bedeutet, dass festgelegt wird, welches Kontrastmittel verwendet wird, welche Stelle des Untersuchungsbereichs die Applikationsstelle ist, welcher Bereich des Untersuchungsobjekts der Kontrollbereich ist, welcher Bereich des Untersuchungsobjekts der Untersuchungsbereich ist, und/oder durch welche Parameter (Größe, Gewicht, Alter, Geschlecht und/oder dergleichen) sich der Untersuchungsbereich auszeichnet. Die spezifizierten Größen gehen dann als Inputparameter in die Berechnung des dritten und/oder vierten Zeitpunkts ein und/oder anhand der spezifizierten Größen wird das jeweilige Modell ausgewählt, das zur Berechnung des dritten und/oder vierten Zeitpunkts geeignet ist.

Es ist beispielsweise denkbar, dass ein Nutzer (z.B. ein Radiologe) in einem MRT-Aufnahme-Gerät den Untersuchungsbereich und/oder den Kontrollbereich festlegt. Dabei kann ein Bild (z.B. eine native MRT-Aufnahme) des Untersuchungsbereich und/oder des Kontrollbereichs erzeugt werden. Das (jeweilige) Bild kann einer automatisierten Bilderkennung zugeführt werden, die konfiguriert ist, die bildhaft dargestellten Objekte zu erkennen. Es wird in einem solchen Fall also die Information, welches der Untersuchungsbereich und/oder welches der Kontrollbereich sein wird, automatisch aus einem oder mehreren Bildern dieser Bereiche extrahiert. Auch die Applikationsstelle kann automatisiert erfasst werden. Zum Beispiel kann eine Kamera ein Bild von der Applikationsstelle (zum Beispiel beim Applizieren) erzeugen und (automatisiert) analysieren, um die Applikationsstelle zu erkennen und die Information über die Applikationsstelle an die Einheiten, die sie zur Berechnung von Zeitpunkten benötigen, weiterleiten.

Zur Ausführung der vorliegenden Erfindung kann eine Vorrichtung und/oder ein System verwendet werden. Bei der erfindungsgemäßen Vorrichtung kann es sich um ein Computersystem handeln, das in einer kommunikativen Verbindung mit einem MRT-Aufnahme-Gerät und/oder einem Injektor stehen kann.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon, ein berührungsempfindliches Display und/oder dergleichen.

Die erfindungsgemäße Vorrichtung umfasst eine Empfangseinheit, eine Steuer- und Recheneinheit und eine Ausgabeeinheit. Die Steuer- und Recheneinheit dient zum einen der Steuerung der Komponenten der Vorrichtung und der Koordinierung der Datenflüsse zwischen den Komponenten der Vorrichtung und zum anderen der Berechnung des dritten und/oder vierten Zeitpunkts anhand von Inputparametern wie beispielsweise des Applikationszeitpunkts und des Kontrollzeitpunkts bzw. der Zeitspanne dazwischen und ggf. anhand weiterer Inputparameter. Die Inputparameter empfängt die erfindungsgemäße Vorrichtung über die Empfangseinheit. Es ist denkbar, dass mehrere und/oder verschiedene Empfangseinheiten vorhanden sind. Inputparameter können beispielsweise durch Eingabe von einem Nutzer über Eingabemittel wie z.B. eine Tastatur empfangen werden. Inputparameter können ferner von einem anderen Gerät wie beispielsweise einem Injektor, einem externen Zeitmesser, einem MRT-Aufnahmegerät, einem separaten Computersystem beispielsweise über ein drahtloses und/oder kabelgebundenes Netzwerk oder eine entsprechende Verbindung empfangen werden. Die Steuer- und Recheneinheit kann ferner konfiguriert sein, Inputgrößen aus einer Datenbank auszulesen, wobei die Datenbank ein Bestandteil des Computersystems sein kann oder mit dem Computersystem über eine Netzwerkverbindung verbunden sein kann.

Die erfindungsgemäße Vorrichtung kann ein Bestandteil eines MRT-Aufnahmegeräts sein oder es kann sich um eine separate Vorrichtung handelt, die mit einem MRT-Aufnahmegerät kommunikativ verbunden ist, so dass die erfindungsgemäße Vorrichtung bei Erreichen des dritten und/oder vierten Zeitpunkts ein Signal an das MRT-Aufnahmegerät übermitteln kann, wobei das Signal das MRT-Aufnahmegerät veranlasst, eine MRT-Aufnahme oder eine zeitliche Sequenz von MRT-Aufnahmen von dem Untersuchungsbereich aufzunehmen.

Das erfindungsgemäße System umfasst einen Injektor, eine Sensoreinheit, eine Steuer- und Recheneinheit und eine Aufnahmeeinheit. Mittels des Injektors wird einem Untersuchungsobjekt zum Applikationszeitpunkt ein Kontrastmittel in Form eines Bolus verabreicht. Mittels der Sensoreinheit wird registriert, wann das verabreichte Kontrastmittel den Kontrollbereich erreicht (der Kontrollzeitpunkt). Die Steuer- und Recheneinheit ermittelt anhand des Applikationszeitpunkts und des Kontrollzeitpunkts den Zeitpunkt, zu dem das Kontrastmittel den Untersuchungsbereich erreichen wird (dritter Zeitpunkt). Die Steuer- und Recheneinheit kann ferner konfiguriert sein, aus dem dritten Zeitpunkt einen vierten Zeitpunkt zu berechnen, der in einem zeitlich definierten Abstand vor oder nach dem dritten Zeitpunkt liegt. Die Aufnahmeeinheit startet zum dritten oder vierten Zeitpunkt die Erzeugung einer MRT-Aufnahme von dem Untersuchungsbereich. Bei der Sensoreinheit und der Applikationseinheit kann es sich um dieselbe Einheit handeln.

Die Erfindung wird nachstehend anhand von Zeichnungen näher erläutert, ohne die Erfindung auf die in den Figuren gezeigten Merkmale oder Merkmalskombinationen beschränken zu wollen.

Es zeigen:

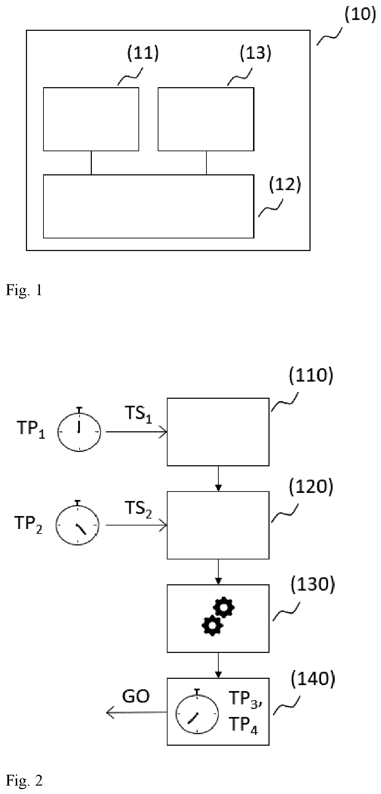

Fig. 1 zeigt schematisch eine Ausführungsform der erfindungsgemäßen Vorrichtung. Die Vorrichtung (10) umfasst eine Empfangseinheit (11), eine Steuer- und Recheneinheit (12) und eine Ausgabeeinheit (13).
Fig. 2 zeigt schematisch in Form eines Ablaufdiagramms die Schritte, die in einer bevorzugten Ausführungsform von der erfindungsgemäßen Vorrichtung vorgenommen werden. Die Schritte sind:
   (110) Empfangen eines ersten Signals (TS₁), wobei das erste Signal (TS₁) einen ersten Zeitpunkt (TP₁) kennzeichnet, wobei zum ersten Zeitpunkt (TP₁) einem Untersuchungsobjekt ein Kontrastmittel in Form eines Bolus verabreicht wird,
   (120) Empfangen eines zweiten Signals (TS₂), wobei das zweite Signal (TS₂) einen zweiten Zeitpunkt (TP₂) kennzeichnet, wobei zum zweiten Zeitpunkt (TP₂) der Bolus einen Kontrollbereich im Untersuchungsobjekt erreicht,
   (130) Berechnen eines dritten Zeitpunkts (TP₃) auf Basis des ersten Zeitpunkts (TP₁) und des zweiten Zeitpunkts (TP₂), wobei der Bolus zum dritten Zeitpunkt (TP₃) einen Untersuchungsbereich im Untersuchungsobjekt erreicht,
   (140) Ausgeben eines Startsignals (GO) zum dritten Zeitpunkt (TP₃) oder zu einem vierten Zeitpunkt (TP₄), wobei der vierte Zeitpunkt (TP₄) in einem definierten zeitlichen Abstand zum dritten Zeitpunkt (TP₃) liegt, wobei das Startsignal (GO) zum Starten der Erzeugung einer MRT-Aufnahme von dem Untersuchungsbereich dient.

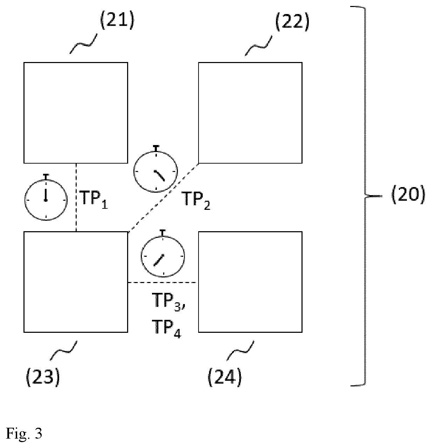

Fig. 3 zeigt schematisch und beispielhaft eine Ausführungsform des erfindungsgemäßen Systems. Das System umfasst einen Injektor (21), eine Sensoreinheit (22), eine Steuer- und Recheneinheit (23) und eine Aufnahmeeinheit (24) für MRT-Aufnahmen.

Über den Injektor (21) wird einem Untersuchungsobjekt zu einem Zeitpunkt TP₁ ein Kontrastmittel verabreicht. Der Injektor (21) ist mit der Steuer- und Recheneinheit (23) verbunden. Es ist denkbar, dass der Injektor (21) zum Zeitpunkt TP₁ durch die Steuer- und Recheneinheit (23) veranlasst wird, Kontrastmittel zu verabreichen. Es ist auch denkbar, dass der Injektor (21) zum Zeitpunkt TP₁ durch eine separate, nicht zwingend zum erfindungsgemäßen System gehörende und in Fig. 3 nicht dargestellte Steuereinheit veranlasst wird, Kontrastmittel zu verabreichen. Ferner ist denkbar, dass der Injektor (21) zum Zeitpunkt TP₁ durch eine zum Injektor (21) gehörende Steuereinheit veranlasst wird, Kontrastmittel zu verabreichen. In jedem Fall wird die Information über den Zeitpunkt TP₁ für die Steuer- und Recheneinheit (23) verfügbar gemacht. Die Sensoreinheit (22) überwacht einen Kontrollbereich. Erreicht das Kontrastmittel den Kontrollbereich, so übermittelt die Sensoreinheit (22) ein Signal an die Steuer- und Recheneinheit (23), wobei das Signal den Zeitpunkt TP₂ angibt, zu dem das Kontrastmittel den Kontrollbereich erreicht hat. Es ist denkbar, dass die Sensoreinheit (22) das Signal nicht direkt an die Steuer- und Recheneinheit (23) übermittelt; es ist denkbar, dass die Sensoreinheit (22) über eine separate, nicht zwingend zum erfindungsgemäßen System gehörende und in Fig. 3 nicht dargestellte Steuereinheit angesteuert wird und das Signal an diese Steuereinheit übermittelt. Die Steuereinheit übermittelt dann die Information über den Zeitpunkt TP₂ an die Steuer- und Recheneinheit (23) des erfindungsgemäßen Systems. Die Steuer- und Recheneinheit (23) ermittelt anhand des Zeitpunkts TP₁ und des Zeitpunkts TP₂ einen Zeitpunkt TP₃, zu dem das Kontrastmittel einen Untersuchungsbereich erreichen wird. Es ist denkbar, dass die Steuer- und Recheneinheit (23) einen Zeitpunkt TP₄ ermittelt, der in einem definierten zeitlichen Abstand zum Zeitpunkt TP₃ liegt. Bei Erreichen des Zeitpunkts TP₃ oder TP₄ veranlasst die Steuer- und Recheneinheit (23) die Aufnahmeeinheit (24) zum Starten einer MRT-Aufnahme des Untersuchungsbereichs. Alternativ übermittelt die Steuer- und Recheneinheit (23) die Information über den Zeitpunkt TP₃ und/oder TP₄ an eine separate Steuereinheit für die Aufnahmeeinheit (24), welche dann wiederum die Aufnahmeeinheit (24) zum Starten der Erzeugung eines MRT-Aufnahme zum Zeitpunkt TP₃ oder TP₄ veranlasst.

## Patentansprüche

1. Vorrichtung umfassend:
- eine Empfangseinheit,
- eine Steuer- und Recheneinheit, und
- eine Ausgabeeinheit
- wobei die Steuer- und Recheneinheit konfiguriert ist, die Empfangseinheit zu veranlassen, ein erstes Signal zu empfangen, wobei das erste Signal einen ersten Zeitpunkt kennzeichnet, wobei zum ersten Zeitpunkt einem Untersuchungsobjekt ein Kontrastmittel in Form eines Bolus verabreicht wird,
- wobei die Steuer- und Recheneinheit konfiguriert ist, die Empfangseinheit zu veranlassen, ein zweites Signal zu empfangen, wobei das zweite Signal einen zweiten Zeitpunkt kennzeichnet, wobei zum zweiten Zeitpunkt der Bolus einen Kontrollbereich erreicht,
- wobei die Steuer- und Recheneinheit konfiguriert ist, auf Basis des ersten Zeitpunkts und des zweiten Zeitpunkts einen dritten Zeitpunkt zu berechnen, wobei der Bolus zum dritten Zeitpunkt einen Untersuchungsbereich erreicht,
- wobei die Steuer- und Recheneinheit konfiguriert ist, die Ausgabeeinheit zu veranlassen, ein Startsignals zum dritten Zeitpunkt und/oder zu einem vierten Zeitpunkt auszugeben, wobei der vierte Zeitpunkt in einem definierten zeitlichen Abstand zum dritten Zeitpunkt liegt.

2. Vorrichtung gemäß Anspruch 1, wobei das Untersuchungsobjekt ein Mensch ist, wobei der Bolus dem Mensch intravenös an einer Applikationsstellen verabreicht wird, wobei der Untersuchungsbereich eine Leber ist oder ein Teil der Leber des Untersuchungsobjekts ist.

3. Vorrichtung gemäß Anspruch 1 oder 2, wobei das Kontrastmittel ein hepatobiliäres Kontrastmittel, vorzugsweise ein Stoffgemisch mit der Gadoxetsäure oder einem Salz der Gadoxetsäure als kontrastverstärkenden Wirkstoff ist.

4. Vorrichtung gemäß einem der Ansprüche 1 bis 3, wobei die Steuer- und Recheneinheit konfiguriert ist, den dritten und/oder vierten Zeitpunkt mittels eines Modells zu berechnen, wobei das Modell in einem überwachten Lernverfahren trainiert worden ist, anhand von Inputparametern den dritten und/oder vierten Zeitpunkt vorherzusagen, wobei die Inputparameter den ersten Zeitpunkt und den zweiten Zeitpunkt und/oder die Zeitspanne zwischen dem ersten und dem zweiten Zeitpunkt umfassen.

5. Vorrichtung gemäß Anspruch 4, wobei das Modell mit Hilfe eines Random-Forest-Verfahrens erstellt worden ist und/oder wobei das Modell ein künstliches neuronales Netz ist oder ein solches umfasst.

6. Vorrichtung gemäß einem der Anspruch 4 oder 5, wobei die Inputparameter ferner einen oder mehreren der folgenden Parameter umfassen:
- Größe und Gewicht des Untersuchungsobjekts
- Alter des Untersuchungsobjekts
- Körperzusammensetzung des Untersuchungsobjekts

7. Vorrichtung gemäß 6, wobei die Inputparameter ferner einen oder mehreren der folgenden Parameter umfassen:
- Menge des applizierten Kontrastmittels
- Art des applizierten Kontrastmittels
- Applikationsstelle
- Kontrollbereich
- Untersuchungsbereich.

8. System umfassend:
- einen Injektor, wobei der Injektor konfiguriert ist, einem Untersuchungsobjekt Kontrastmittel in Form eines Bolus zu einem ersten Zeitpunkt zu verabreichen,
- eine Sensoreinheit, wobei die Sensoreinheit konfiguriert ist, das Eintreffen des Bolus in einem Kontrollbereich zu einem zweiten Zeitpunkt zu erfassen,
- eine Steuer- und Recheneinheit, wobei die Steuer- und Recheneinheit konfiguriert ist, auf Basis des ersten Zeitpunkts und des zweiten Zeitpunkts einen dritten Zeitpunkt zu berechnen, wobei der Bolus zum dritten Zeitpunkt einen Untersuchungsbereich erreicht,
- eine Aufnahmeeinheit für MRT-Aufnahmen, wobei die Aufnahmeeinheit konfiguriert ist, die Erzeugung einer MRT-Aufnahme von dem Untersuchungsbereich zum dritten Zeitpunkt oder zu einem vierten Zeitpunkt zu starten, wobei der vierte Zeitpunkt in einem definierten zeitlichen Abstand zum dritten Zeitpunkt liegt.

9. System gemäß Anspruch 8, wobei die Steuer- und Recheneinheit konfiguriert ist, bei Erreichen des dritten oder vierten Zeitpunkts ein Signal an die Aufnahmeeinheit zu übermitteln, wobei das Signal die Aufnahmeeinheit veranlasst, mindestens eine MRT-Aufnahme von dem Untersuchungsbereich zu erzeugen.

10. System gemäß einem der Ansprüche 8 oder 9, umfassend eine Vorrichtung gemäß einem der Ansprüche 1 bis 7, einen Injektor, eine Sensoreinheit und eine Aufnahmeeinheit.

11. Verfahren umfassend die Schritte
- Empfangen eines ersten Signals, wobei das erste Signal einen ersten Zeitpunkt kennzeichnet, wobei zum ersten Zeitpunkt einem Untersuchungsobjekt ein Kontrastmittel in Form eines Bolus verabreicht wird,
- Empfangen eines zweiten Signals, wobei das zweite Signal einen zweiten Zeitpunkt kennzeichnet, wobei zum zweiten Zeitpunkt der Bolus einen Kontrollbereich erreicht,
- Berechnen eines dritten Zeitpunkts auf Basis des ersten Zeitpunkts und des zweiten Zeitpunkts, wobei der Bolus zum dritten Zeitpunkt einen Untersuchungsbereich erreicht,
- Ausgeben eines Startsignals zum dritten Zeitpunkt und/oder zu einem vierten Zeitpunkt, wobei der vierte Zeitpunkt in einem definierten zeitlichen Abstand zum dritten Zeitpunkt liegt,
- Starten der Erfassung einer MRT-Aufnahme von dem Untersuchungsbereich zum dritten oder vierten Zeitpunkt.

12. Verfahren gemäß Anspruch 11, wobei das Erreichen des Kontrollbereichs durch den Bolus mittels MR-Fluoroskopie detektiert wird.

13. Verfahren gemäß einem der Ansprüche 11 oder 12, wobei der Kontrollbereich so gewählt wird, dass eine erste Zeitspanne zwischen dem ersten Zeitpunkt und dem zweiten Zeitpunkt größer ist als eine zweite Zeitspanne zwischen dem zweiten Zeitpunkt und dem dritten und/oder vierten Zeitpunkt, wobei die erste Zeitspanne vorzugsweise mindestens das Anderthalbfache bis Zweifache der zweiten Zeitspanne beträgt.

14. Computerprogrammprodukt umfassend einen Datenträger, auf dem ein Computerprogramm gespeichert ist, wobei das Computerprogramm in einen Arbeitsspeicher eines Computers geladen werden kann und dort den Computer dazu veranlasst, folgende Schritte ausführen:
- Empfangen eines ersten Signals, wobei das erste Signal einen ersten Zeitpunkt kennzeichnet, wobei zum ersten Zeitpunkt einem Untersuchungsobjekt ein Kontrastmittel in Form eines Bolus verabreicht wird,
- Empfangen eines zweiten Signals, wobei das zweite Signal einen zweiten Zeitpunkt kennzeichnet, wobei zum zweiten Zeitpunkt der Bolus einen Kontrollbereich erreicht,
- Berechnen eines dritten Zeitpunkts auf Basis des ersten Zeitpunkts und des zweiten Zeitpunkts, wobei der Bolus zum dritten Zeitpunkt einen Untersuchungsbereich erreicht,
- Ausgeben eines Startsignals zum dritten Zeitpunkt und/oder zu einem vierten Zeitpunkt, wobei der vierte Zeitpunkt in einem definierten zeitlichen Abstand zum dritten Zeitpunkt liegt,
- Starten der Erfassung einer MRT-Aufnahme von dem Untersuchungsbereich zum dritten oder vierten Zeitpunkt.

15. Kontrastmittel zur Verwendung in einem MRT-Verfahren, wobei das MRT-Verfahren die folgenden Schritte umfasst:
- Applizieren des Kontrastmittels in Form eines Bolus zu einem ersten Zeitpunkt,
- Erfassen des Eintreffens des Bolus in einem Kontrollbereich,
- Berechnen eines Startzeitpunkts anhand der Zeitspanne zwischen dem Applizieren des Kontrastmittels und dem Eintreffen des Bolus in dem Kontrollbereich,
- Starten der Erzeugung einer MRT-Aufnahme von einem Untersuchungsbereich zum Startzeitpunkt
wobei es sich bei dem Kontrastmittel vorzugsweise um ein hepatobiliäres Kontrastmittel, besonders bevorzugt um einen Stoff oder ein Stoffgemisch mit der Gadoxetsäure oder einem Salz der Gadoxetsäure als kontrastverstärkenden Wirkstoff, am meisten bevorzugt um das Dinatriumsalz der Gadoxetsäure handelt.

16. Kit umfassend ein Kontrastmittel und das Computerprogrammprodukt gemäß Anspruch 14, wobei es sich bei dem Kontrastmittel vorzugsweise um ein hepatobiliäres Kontrastmittel, besonders bevorzugt um einen Stoff oder ein Stoffgemisch mit der Gadoxetsäure oder einem Salz der Gadoxetsäure als kontrastverstärkenden Wirkstoff, am meisten bevorzugt um das Dinatriumsalz der Gadoxetsäure handelt.
